## Europäisches Patentamt

## European Patent Office

(11) Veröffentlichungsnummer: **0 148 193**
**B1**

## Office européen des brevets

(19)

(12) **EUROPÄISCHE PATENTSCHRIFT**

(45) Veröffentlichungstag der Patentschrift:
12.08.87

(51) Int. Cl.⁴: **C 12 Q 1/36**

(21) Anmeldenummer: **84901763.7**

(22) Anmeldetag: **28.04.84**

(86) Internationale Anmeldenummer:
**PCT/EP 84/00127**

(87) Internationale Veröffentlichungsnummer:
**WO 84/04331 (08.11.84 Gazette 84/26)**

(54) **VERFAHREN UND REAGENS ZUR BESTIMMUNG VON "ANGIOTENSIN CONVERTING ENZYME".**

(30) Priorität: **03.05.83 BE 210688**

(43) Veröffentlichungstag der Anmeldung:
**17.07.85 Patentblatt 85/29**

(45) Bekanntmachung des Hinweises auf die Patenterteilung:
**12.08.87 Patentblatt 87/33**

(84) Benannte Vertragsstaaten:
**AT BE CH DE FR GB LI LU NL SE**

(56) Entgegenhaltungen:
**GB-A-2 060 646**
**US-A-4 108 726**
**US-A-4 334 041**

*Chemical Abstracts, Vol. 92, No. 23, 9 June 1980, (Columbus, Ohio, US), K.A. Holm "Automated colorimetric determination of acid proteinase activity in fermentation samples using a trinitrobenzenesulfonic acid reagent", see page 236, abstract 193223f*
*Chemical Abstracts, Vol. 92, No. 23, 9 June 1980, (Columbus, Ohio, US), D.X. Chu et al.: "A colorimetric method for the determination of urokinase with 2,4,6 trinitrobenzene-1-sulfonic acid", see page 239, abstract 193253r*
*Chemical Abstracts, Vol. 89, No. 19, 6 November 1978 (Columbus, Ohio, US), W. Reisner: "Tegewa method for the determination of the activity of*

(73) Patentinhaber: **Boehringer Mannheim GmbH, Sandhofer Strasse 116, D-6800 Mannheim 31 (DE)**

(72) Erfinder: **SCHARPE, Simon, L., Kerkhofstraat 7, B-9380 Wieze (BE)**
Erfinder: **VAN SANDE, Marc, E., Donkseinde 40, B-2130 Brasschaat (BE)**
Erfinder: **NEELS, Hugo, M., Prins Leopoldlaan 2 - bus 7, B-2600 Berchem (BE)**

(56) Entgegenhaltungen: (Fortsetzung)
*proteolytic enzymes with trinitrobenzenesulfonic acid (TNBA)", see page 216, abstract 159232m*
*J.D. ROBERTS et al. "Basic Principles of Organic Chemistry", 1965, W.A. Benjamin, New York, Amsterdam, Kapitel 20, pages 716-717, see page 716, lines 10-33; page 717, lines 1-4; reaction*

**0 148 193**

**Beschreibung**

Die Erfindung betrifft ein Verfahren und ein Reagens zur Bestimmung der Aktivität von "Angiotensin Converting Enzyme", im folgenden als ACE abgekürzt.

Das Angiotensin I umwandelnde Enzym (E. C. 3.4.15.1, Dipeptidyl Carboxy-Peptidase, ACE, Kinase II) ist ein membrangebundenes Glycoprotein, das ursprünglich in den Endothelial-Zellen der pulmonaren Kapillaren und in der Nierenrinde vorkommt. ACE spaltet das Dipeptid L-Histidyl-L-Leucin vom Angiotensin I ab, um das gefäßverengende Angiotensin II freizusetzen. Außerdem katalysiert ACE den Abbau des gefäßerweiternden Nonapeptids Bradykinin durch aufeinanderfolgende Freisetzung von L-Phenylalanyl-L-Arginin und L-Seryl-L-Prolin. Im Hinblick auf diese Aktivität gegenüber Bradykinin nennt man ACE auch Kinase II. Somit nimmt ACE eine wichtige Stellung sowohl im Renin-Angiotensin-Aldosteron-System als auch im Kallikrein-Bradykinin-System ein. Vom biochemischen Standpunkt aus gesehen stehen beide Systeme in engem Zusammenhang mit den proteolytischen Reaktionsschritten der Blutgerinnung, Fibrinolyse und Komplement-Aktivierung.

ACE ist ein Metallo-Protein mit einem Molekulargewicht von 130.000, das aus einer einzigen Kette besteht. Obwohl ACE eine stärkere Affinität zur Bradykinin als zu angiotensin I besitzt, wird es gewöhnlich als Angiotensin umwandelndes Enzym ("Angiotensin Converting Enzyme") bezeichnet. ACE kann sein eigenes Spaltprodukt, Angiotensin II, nicht weiter hydrolisieren; vermag jedoch mehrere Aminosäuresequenzen zu spalten.

Angiotensin I Converting Enzyme wird als ein unentbehrlicher Parameter für den Nachweis klinisch aktiver Sarcoidose betrachtet. ACE wird auch benutzt, um die Effektivität der Steroid-Therapie dieser Krankheit zu überwachen.

In jüngster Zeit sind mehrere ACE-Bestimmungsmethoden veröffentlicht worden, beispielsweise in der Deutschen Offenlegungsschrift 30 34 618.

Der der Anmelderin bekannte Stand der Technik wird in folgender Zusammenstellung aufgeführt:

1. Liebermann J., Nosal A., Schlessner L. A., Sastre-Foken A. "Serum Angiotensin-Converting Enzyme for diagnosis and therapeutic evaluation of sarcoidosis", Am. Rev. Respir. Dis. 120, 329 - 335 (1979).

2. Liebermann J. "Evaluation of serum angiotensin converting enzyme (ACE) level in sarcoidosis", Am. J. Med. 59, 365 - 372 (1975).

3. Chiu A. T., Ryan J. W., Ryan U. S., Dorer F. E. "A sensitive radiochemical assay for angiotensin-converting enzyme (kinase II)", Biochem. J. 149, 297 - 300 (1975) (siehe auch US-P 4 260 682).

4. Rohrbach M. S., Deremee R. A. "Serum angiotensin converting enzyme activity in sarcoidosis as measured by a simple radiochemical assay", Am. Rev. Respir. Dis. 119, 761 - 767 (1979).

5. Kasahara Y., Ashihara Y. "Colorimetry of angiotensin-I converting enzyme activity in serum", Clin. Chem. 27, 1922 - 1925 (1981).

6. Lanzillo J. J., Fanburg B. L. "Development of competitive enzyme immunoassays for human serum. Angiotensin-I-converting enzyme. A comparison of our assay configurations. Anal. Biochem. 126, 156 - 164 (1982).

7. Hurst P. L., Lovell-Smith C. J. "Optimized assay for serum angiotensin-converting enzyme activity", Clin. Chem. 27, 2048 - 2052 (1981).

8. Neels H. M., Scharpé S. L., van Sande M. E., et al. "Improved micromethod for assay of serum angiotensin converting enzyme", Clin. Chem. 28, 1352 - 1355 (1982).

9. Depierre D., Roth M. "Flurimetric determination of dipeptidyl carboxy-peptidase", Enzyme 19, 65 - 70 (1975).

10. Friedland J., Silverstein E. "A sensitive fluorimetric assay for serum angiotensin-converting enzyme" Am. J. Clin. Pathol. 66, 416 - 424 (1976).

Die in der oben erwähnten Literatur beschriebenen Methoden weisen jedoch zahlreiche Nachteile und Mängel auf, so z. B. mehrere Extraktions- und Wiederauflösungs-Schritte (2), die Notwendigkeit, radioaktive Substrate einzusetzen (3, 4), der Einsatz von komplexen und teuren enzymatischen Hilfsreaktionen (5,6), der Gebrauch von schädlichen Reagenzien (7) und der Bedarf an speziellen Instrumenten, wie z. B. Flüssigkeitsscintillationszähler (3, 4), Hochdruck-flüssigkeitschromatographische Ausstattung (8) oder Fluorometer (9,10).

US-C-4 335 041 beschreibt Verfahren zur Bestimmung von A.C.E mittels radioaktiv markierter Substrate. Als Substrat wird u.a. Hippuryl-Histidyl-Leucin in Gegewart hoher Sulfationenkonzentrationen eingesetzt. Das durch die enzymatische Spattung entstandene radioaktiv markierte Hip-Fragment wird durch Extraktion mit einem organischen Lösungsmittel isoliert und in einem Szintillationszähler gemessen.

US-C-4 108 726 beschriebt, daß als Substrat Hippuryl-Histidyl-Leucin eingesetzt und das resultierende Dipeptid Histdyl-Leucin nach Umsetzung mit Ortho-Phthaldialdehyd flourometrisch bestimmt wird. Bei Einsatzt eines großen Überschusses an ortho-Phthaldialdehyd war es möglich, das Reaktionsprodukt ohne vorherige Abtrennung zu messen.

Aufgabe der Erfindung war es, eine ACE-Bestimmungsmethode bereitzustellen, die preiswerte Reagenzien benötigt, die bereits mit Hilfe eines einfachen Photometers durchgeführt werden kann und die daher für einfache Anwendungen in klinischen Laboratorien geeignet ist.

Diese Aufgabe wird durch das erfindungsgemäße Verfahren gelöst, das in den Ansprüchen näher charakterisiert ist.

Die Bestimmung von ACE gemäß der Erfindung wird zweckmäßigerweise in folgenden Schritten

2

durchgeführt:

1. Man mischt eine ACE enthaltende Probe, z. B. eine Flüssigkeit, mit synthetischen Substraten, wie z. B. Hippuryl-Glycyl-Glycin oder Hippuryl-L-Histidyl-L-Leucin, und deproteinisiert, vorzugsweise nach der Folin-Wu-Methode oder durch Dialyse, so daß man freie, nicht ausgefällte Peptid-Derivate, wie z. B. Glycyl-Glycin oder L-Histidyl-L-Leucin in der Lösung erhält.

2. Man bestimmt, mittels Photometrie, die Peptid-Derivate, die man nach Wechselwirkung von ACE mit den oben angegebenen Substraten gemäß Punkt 1. erhält, durch Arylierung mittels Nitrobenzol-Verbindungen, wie z. B. Trinitrobenzolsulfonsäure oder 1-Fluor-2,4-dinitrobenzol.

Das ACE-Substrat wird üblicherweise in einer Konzentration von 10 bis 100 mmol/l, vorzugsweise von 20 bis 50 mmol/l eingesetzt. Zweckmäßigerweise wird es der ACE-enthaltenden Probe in Form einer gepufferten Lösung zugemischt. Der pH-Wert dieser Puffer-Substrat-Lösung sollte üblicherweise im schwach alkalischen Bereich, zweckmäßig bei pH 7 bis 9, besonders bevorzugt bei ca. 8.0, liegen. Als Puffer-Substanzen sind solche Puffer geeignet, deren Pufferwirkung in diesem Bereich liegen.

Der Deproteinisierungs-Schritt kann nach üblichen, dem Fachmann bekannten Methoden durchgeführt werden. Besonders geeignet hat sich die Methode nach Folin-Wu oder die Deproteinisierung durch Dialyse erwiesen. Zweckmäßigerweise versetzt man die Reaktionslösung zur Deproteinisierung mit einer Wolframat-Lösung, beispielsweise Natrium-Wolframat, und verdünnter Schwefelsäure.

Nach dem Deproteinisierungs-Schritt wird von den ausgefällten Proteinen abzentrifugiert und im Überstand die abgespaltenen, noch in Lösung befindlichen Dipeptide nachgewiesen. Der Nachweis dieser Dipeptide wird in üblicher, dem Fachmann bekannter Weise geführt. Für das erfindungsgemäße Verfahren hat sich besonders bewährt, die Dipeptide mit Nitrobenzol-Derivaten zu arylieren und die arylierten Produkte anhand ihrer charakteristischen Absorption photometrisch zu bestimmen.

Ein weiterer Gegenstand der Erfindung ist ein Reagenz zur Bestimmung der Aktivität von ACE, das dadurch gekennzeichnet ist, daß es als ACE-Substrat Hippuryl-Glycyl-Glycin oder Hippuryl-L-Histidyl-L-Leucin, zusätzlich Substanzen zur Durchführung des Deproteinisierungs-Schrittes und ein oder mehrere Nitrobenzol-Derivate zur Bestimmung der Peptid-Spaltprodukte, sowie gegebenenfalls weitere erforderliche Hilfsstoffe enthält. Die Konzentration der ACE-Substrate in dem Reagens beträgt zweckmäßigerweise 10 bis 100 mmol/l, besonders bevorzugt 20 bis 50 mmol/l. Als Hilfsstoffe kommen vor allem in Frage: Puffersubstanzen, Stabilisierungsmittel, oberflächenaktive Mittel usw.

Die Empfindlichkeit und Reproduktionsfähigkeit der erfindungsgemäßen Methode sind sehr gut. 10 µl Serum genügen, um sehr zuverlässige Ergebnisse zu erhalten. Dies wird durch sehr geringe Variations-Koeffizienten bestätigt.

Die folgenden Parameter wurden ermittelt:

Abwiechungen innerhalb eines Tages:

2,2 % für eine ACE-Aktivität von 200 U/l (Enheit/Liter)

1,6 % für eine ACE-Aktivität von 945 U/l

Abweichungen von Tag zu Tag:

2,3 % für eine ACE-Aktivität von 194 ul

2,7 % für eine ACE-Aktivität von 944 ul

Charakteristisch für die Erfindung sind die gute Reproduzierbarkeit der Testergebnisse, die nach der erfindungsgemäßen Methode erhalten worden sind und die Einfachheit der Durchführung der beanspruchten Methode, wodurch deren universelle Anwendungsmöglichkeit erheblich gesteigert wird.

### A. Für das erfindungsgemäße Verfahren werden die folgenden Reagenzien verwendet:

1. Puffer-Substrat-Lösung bestehend aus:

a) 4-(2-Hydroxyethyl)-1-piperazinethansulfonsäure: 50 mmol/l

b) Natriumchlorid: 300 mmol/l

c) Natriumsulfat: 400 mmol/l

d) Hippuryl-Glycyl-Glycin: 30 mmol/l.

Mit Natronlauge auf pH-Wert 8.15 bei 25° C eingestellt.

2. Wolframat-Lösung: 100 g/l

3. Schwefelsäure: 0,33 mol/l

4. Glycyl-Glycin-Lösung. 0,020 mol/l

5. Trinitrobenzolsulfonsäure-Lösung: 60 mmol/l

6. Borat-Puffer: 100 mmol/l

## 0 148 193

**B. Beschreibung der Methode**

| Ablauf | Serumprobe | Serum Blindwert | Standard |
|---|---|---|---|
| | ($\mu$l) | ($\mu$l) | ($\mu$l) |
| 1. Folgende Lösungen werden gemischt: | | | |
| Puffer-Substrat-Lösung | 100 | 100 | 100 |
| Wolframat-Lösung, 100 g/l | - | 100 | 100 |
| Schwefelsäure 0,33 mol/l | - | 100 | 100 |
| 2. Bei 37°C wird 5 Minuten vorinkubiert. Dann wird zugefügt: | | | |
| Serum | 10 | 10 | 10 |
| Glycyl-Glycin-Lösung, 20 mmol/l | - | - | 10 |
| 3. Bei geschlossenem Gefäß wird 30 Minuten bei 37°C inkubiert. Dann wird zugefügt: | | | |
| Wolframat-Lösung, 100 g/l | 100 | - | - |
| Schwefelsäure, 0,33 mol/l | 100 | - | - |
| 4. Es wird unmittelbar vermischt und dann zugefügt: | | | |
| Wasser | 1000 | 1000 | 990 |
| 5. Es wird erneut gemischt; danach zentrifugiert (annähernd 2000 x g). Danach werden folgende Mischungen zubereitet: | | | |
| Überstand | 750 | 750 | 750 |
| Borat-Puffer | 1000 | 1000 | 1000 |
| Trinitrobenzolsulfon-säure-Lösung | 50 | 50 | 50 |

6. Die nach "5." erhaltene Flüssigkeit wird bei 37°C 15 Minuten oder bei Raumtemperatur mindestens 30 Minuten stehengelassen. Die Absorptionen ($\Delta$E) bei 420 nm werden gemessen, z. B. mittels eines Photometers. Die Absorption der Serumprobe wird mit dem Serumblindwert verglichen.

Aus der beobachteten Absorptionsdifferenz läßt sich in üblicher Weise die ACE-Aktivität ermitteln:
ACE = [$\Delta$(Serumprobe) -$\Delta$E(Serumblindwert)] x 670.
Die Aktivität in Einheit pro Liter entspricht:

$$\frac{\Delta A \times 10^6 \times 1310 \times 1,8 \times 10^{-3}}{15650 \times 1 \times 30 \times 10^{-5} \times 750}$$

In dieser Gleichung sind die folgenden Parameter eingesetzt:
- molarer Absorptions-Koeffizient von Trinitrophenyl-Glycyl-Glycin = 15.650 $1.mol^{-1}.cm^{-1}$
- der optische Durchmesser der Küvette = 1 cm
- Inkubationszeit = 30 Minuten
- Menge des untersuchten Serums = $10^{-5}$ l
- Umwandlungsfaktor von mol/l zu ACE unit/l = = $10^6$
- Verhältnis der gebildeten Spaltprodukte, die aryliert werden = 750/1310
- erreichte Volumenverdünnung = 1.8 x $10^{-3}$ 1.

**C. Weiteres Beispiel für die erfindungsgemäße Methode**

1. Puffer-Substrat-Lösung
In einen 20,0-ml-Meßkolben werden folgende Zusätze gegeben (Endkonzentrationen in Parenthese):
0,238 g 4-(2-Hydroxyethyl)-1-piperazinethansulfonsäure (50 mmol/l),
0,351 g Natriumchlorid (300 mmol/l),
1,136 g Natriumsulfat (400 mmol/l),
0.179 g Hippuryl-Glycyl-Glycin (30 mmol/l).
Es wird die entsprechende Menge destilliertes Wasser und 50 $\mu$l gesättigte Natronlauge zugegeben. Sobald sich alles gelöst hat, wird mit Natronlauge auf pH-Wert 8.15 bei 25°C (8.0 bei 37°C) eingestellt.

Die so hergestellte Lösung wird in 100- µl-Portionen in Teströhrchen (75 mm x 8 mm) gefüllt und verschlossen bei -25°C bis zum Gebrauch aufbewahrt.

2. Borat-Puffer-Lösung

9,53 g (100 mmol/l) $Na_2B_4O_7.10H_2O$ werden in 250 ml destilliertes Wasser aufgelöst. Mit Natronlauge wird auf pH-Wert 9,6 eingestellt.

3. 2,4,6-Trinitrobenzolsulfonsäure-Lösung

406 mg 2,4,6-Trinitrobenzolsulfonsäure werden in 20 ml destilliertem Wasser gelöst und bei -25°C aufbewahrt.

4. Spectrophotometrische Messungen

10 µl Serum und 100 µl der oben genannten Puffer-Substrat-Lösung werden 30 Minuten bei 37°C inkubiert. Die Reaktion wird durch aufeinanderfolgende Zugabe von 100 µl Natrium-Wolframat (100 g/l) und 100 µl verdünnte Schwefelsäure (0,33 mol/l) gestoppt.

Die Lösung wird für 10 Sekunden geschüttelt. Danach werden 1000 µl Wasser zugegeben. Nach dem Mischen wird die Lösung 10 Minuten lang bei 2000 x g zentrifugiert. Zu 750 µl Überstand werden 1000 µl Borat-Puffer-Lösung und anschließend 50 µl 2,4,6-Trinitrobenzolsulfonsäure-Lösung gegeben und vermischt. Das erhaltene Gemisch wird 15 Minuten bei 37°C stehengelassen. Danach wird die Absorption gemessen und mit einem Serumblindwert verglichen. Diesen erhält man durch Messen einer Probelösung, die man durch Zugabe von Deproteinisierungszusätzen (Natrium-Wolframat und Schwefelsäure zu der Puffer-Substrat-Lösung, bevor das Serum zugegeben wird, vorbereitet.

Das Verfahren kann zwischen Deproteinisierung und Arylierung für einen längeren Zeitraum (bis zu 3 Tagen) unterbrochen werden. Die photometrischen Meßergebnisse können in U/l umgerechnet werden, z. B. mittels oben genannter Gleichung.

Die erfindungsgemäße Methode kann auch bei menschlichen biologischen Materialien angewandt werden, z. B. Spermien und Samenflüssigkeit, Prostatagewebe, Testisgewebe, Corneagewebe, Macrophagen und Monozyten, die eine bemerkenswerte ACE-Aktivität aufweisen.

Die Aktivität dieses Enzyms kann besonders durch Inhibitoren, wie (2S)-1-(2S)-3-Mercapto-2-methylpropanoyl-prolin, N-1-S-1-Carboxy-3-phenylpropyl-L-alanyl-L-prolin, 3-(Mercaptomethyl)-2-oxo-1-piperidin-essigsäure N-Cyclohexylcyclohexamin-Salz, 4R-3-(2S)-3-Mercapto-2-methyl-propanoyl-4-thiazolidin-carbonsäure, gehemmt werden. Diese Hemmung kann für diagnostische und therapeutische Anwendungen auf medizinischem Gebiet ausgenutzt werden, beispielsweise zum Auslösen, Verhindern oder Unterbrechen der Schwangerschaft zum Unschädlichmachen oder Abbauen von vasoaktiven Substanzen oder zur chemischen Ermittlung und Diagnose von Krankheiten der oben genannten Gewebe.

Es ist offensichtlich, daß die Erfindung nicht auf das vorstehend Beschriebene beschränkt ist. Die Beschreibung und die Beispiele sollen nur zur Veranschaulichung der Erfindung dienen. Es ist selbstverständlich, daß andere, äquivalente Ausführungsformen mit umfaßt sein sollen.

**Patentansprüche**

1. Verfahren zur Bestimmung der Aktivität von "Angiotensin-Converting Enzyme" (ACE), dadurch gekennzeichnet, daß eine ACE-enthaltende Probe mit einem synthetischen Substrat, ausgewählt aus Hippuryl-Glycyl-Glycin und Hippuryl-L-Histidyl-L-Leucin, vermischt, anschließend deproteinisiert und das freigesetzte, in Lösung befindliche Glycyl-Glycin bzw. L-Histidyl-L-Leucin durch Arylierung mit Hilfe von Nitrobenzol-Derivaten und anschließende photometrische Messung der arylierten Produkte bestimmt wird.

2. Verfahren gemäß anspruch 1, dadurch gekennzeichnet, daß das synthetische Substrat in einer Konzentration von 10 bis 100 mmol/l eingesetzt wird.

3. Verfahren gemäß Anspruch 1, dadurch gekennzeichnet, daß die Arylierung mit Hilfe von Trinitrobenzolsulfonsäure oder 1-Fluoro-2,4-dinitrobenzol durchgeführt wird.

4. Verfahren gemäß einem der Ansprüche 1 bis 3, dadurch gekennzeichnet, daß der Deproteinisierungsschritt nach der Folin-Wu-Methode oder durch Dialyse erfolgt.

5. Reagenz zur Bestimmung der Aktivität von "Angiotensin-Converting Enzyme", dadurch gekennzeichnet, daß es als ACE-Substrat Hippuryl-Glycyl-Glycin oder Hippuryl-L-Histidyl-L-Leucin, zusätzlich Substanzen für die Durchführung eines Deproteinisierungs-Schrittes und ein oder mehrere Nitrobenzol-Derivate zur Bestimmung der Dipeptid-Spaltprodukte sowie zusätzliche Hilfsstoffe enthält.

6. Reagenz gemäß Anspruch 5, dadurch gekennzeichnet, daß das ACE-Substrat in einer Konzentration von 10 bis 100 mmol/l enthalten ist.

7. Reagenz gemäß Anspruch 5 oder 6, dadurch gekennzeichnet, daß es als zusätzliche Hilfsstoffe Puffer, Stabilisierungsmittel oder/und oberflächenaktive Mittel enthält.

**0 148 193**

### Claims

1. Process for the determination of the activity of "angiotensin converting enzyme" (ACE), characterised in that an ACE-containing sample is mixed with a synthetic substrate selected from hippurylglycylglycine and hippuryl-L-histidyl-L-leucine, subsequently deproteinised and the liberated glycylglycine or L-histidyl-L-leucine present in solution is determined by arylation with the help of nitrobenzene derivatives and subsequent photometric determination of the arylated products.

2. Process according to claim 1, characterised in that the synthetic substrate is used in a concentration of 10 to 100 mmol/l.

3. Process according to claim 1, characterised in that the arylation is carried out with the help of trinitrobenzene-sulphonic acid or of 1-fluoro-2,4-dinitrobenzene.

4. Process according to one of claims 1 to 3, characterised in that the deproteinisation step takes place according to the Folin-Wu method or by dialysis.

5. Reagent for the determination of the activity of "angiotensin converting enzyme", characterised in that as ACE substrate it contains hippurylglycylglycine or hippuryl-L-histidyl-L-leucine, additional substances for the carrying out of a deproteinising step and one or more nitrobenzene derivatives for the determination of the dipeptide fission products, as well as additional adjuvants.

6. Reagent according to claim 5, characterised in that the ACE substrate is present in a concentration of 10 to 100 mmol/l.

7. Reagent according to claim 5 or 6, characterised in that, as additional adjuvants, it contains buffers, stabilising agents and/or surface-active agents.

### Revendications

1. Procédé pour la détermination de l'activité de l'enzyme de conversion de l'angiotensine (ACE), caractérisé par le fait que l'on mélange un échantillon contenant de l'ACE à un substrat synthétique choisi entre l'hippuryl-glycyl-glycine et l'hippuryl-L-histidyl-L-leucine, qu'ensuite on déprotéinise et que l'on détermine la glycyl-glycine ou la L-histidyl-L-leucine libérée, qui se trouve en solution, par arylation à l'aide de dérivés de nitrobenzène et ensuite mesure photométrique des produits arylés.

2. Procédé selon la revendication 1, caractérisé par le fait que l'on utilise le substrat synthétique à une concentration de 10 à 100 mmol/l.

3. Procécé selon la revendication 1, caractérisé en ce que l'on effectue l'arylation à l'aide d'acide trinitrobenzénesulfonique ou de 1-fluoro-2,4-dinitrobenzène.

4. Procédé selon l'une des revendications 1 à 3, caractérisé par le fait que l'étape de déprotéinisation s'effectue selon la méthode Folin-Wu ou par dialyse.

5. Réactif pour la détermination de l'activité d'enzyme de conversion d'angiotensine, caractérisé en ce qu'il contient comme substrat d'ACE l'hippuryl-glycyl-glycine ou l'hippuryl-L-histidyl-L-leucine, plus des substances pour l'éxécution d'une étape de déprotéinisation et un ou plusieurs dérivés de nitrobenzène pour la détermination des produits de coupure dipeptides ainsi que des adjuvants supplémentaires.

6. Réactif selon la revendication 5, caractérisé en ce que le substrat d'ACE est contenu à une concentration de 10 à 100 mmol/l.

7. Réactif selon l'une des revendications 5 et 6, caractérisé en ce qu'il contient comme adjuvants supplémentaires des tampons, stabilisants et/ou surfactifs.